(19) Europäisches Patentamt
European Patent Office
Office européen des brevets

(11) Veröffentlichungsnummer : **0 127 862**
**B1**

(12) # EUROPÄISCHE PATENTSCHRIFT

(45) Veröffentlichungstag der Patentschrift : **08.07.87**

(21) Anmeldenummer : **84106031.2**

(22) Anmeldetag : **26.05.84**

(51) Int. Cl.$^4$ : **G 03 G 5/09**, G 03 G 5/06, C 07 D263/56, C 07 D277/64, C 09 B 23/12

(54) **Elektrophotographisches Aufzeichnungsmaterial.**

(30) Priorität : **31.05.83 DE 3319654**

(43) Veröffentlichungstag der Anmeldung : **12.12.84 Patentblatt 84/50**

(45) Bekanntmachung des Hinweises auf die Patenterteilung : **08.07.87 Patentblatt 87/28**

(84) Benannte Vertragsstaaten : **DE FR GB NL**

(56) Entgegenhaltungen :
**DE-A- 1 058 836**
**DE-A- 3 141 554**
**DE-B- 1 772 318**
**DE-B- 2 526 720**
**DE-C- 704 141**
**US-A- 3 958 991**

(73) Patentinhaber : **HOECHST AKTIENGESELLSCHAFT**
**Postfach 80 03 20**
**D-6230 Frankfurt am Main 80 (DE)**

(72) Erfinder : **Franke, Werner, Dr. Dipl.-Chem.**
**Wendelsteinstrasse 58**
**D-6200 Wiesbaden (DE)**

**EP 0 127 862 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein elektrophotographisches Aufzeichnungsmaterial, bestehend aus einem elektrisch leitenden Schichtträger und mindestens einer photoleitfähigen Schicht, enthaltend organischen Photoleiter, Sensibilisierungsfarbstoff, Bindemittel und übliche Zusätze.

Es ist bekannt (DE-PS 10 58 836, entsprechend US-PS 3 189 447), für die elektrophotographische Reproduktion organische Photoleiter zu verwenden, deren spektrale Empfindlichkeit in der Regel im Bereich des langwelligen UV-Lichtes von etwa 350 bis 450 nm liegt.

Zur Erweiterung des spektralen Empfindlichkeitsbereiches bis etwa 650 nm ist es bekannt, die verschiedensten Farbstoffe unterschiedlicher Verbindungsklassen als Sensibilisatoren zu verwenden. Als wirksam seien beispielsweise die in den Farbstofftabellen von Schultz (7. Auflage, 1. Band, 1931) aufgeführten Farbstoffe genannt:

Triarylmethanfarbstoffe, wie
- Brillantgrün (Nr. 760, S. 314),
- Victoriablau B (Nr. 822, S. 347),
- Methylviolett (Nr. 783, S. 327),
- Kristallviolett (Nr. 785, S. 329),
- Säureviolett 6B (Nr. 831, S. 351);

Xanthenfarbstoffe, und zwar Rhodamine, wie
- Rhodamin B (Nr. 864, S. 365),
- Rhodamin 6G (Nr. 866, S. 366),
- Rhodamin G extra (Nr. 865, S. 366),
- Sulforhodamin B (Nr. 863, S. 364) und
- Echtsäureeosin G (Nr. 870, S. 368),

sowie Phthaleine, wie
- Eosin S (Nr. 883, S. 375),
- Eosin A (Nr. 881, S. 374),
- Erythrosin (Nr. 886, S. 376),
- Phloxin (Nr. 890, S. 378),
- Rose bengale (Nr. 889, S. 378) und
- Fluorescein (Nr. 880, S. 373);

Thiazinfarbstoffe, wie
- Methylenblau (Nr. 1 038, S. 449);

Acridinfarbstoffe, wie
- Acridingelb (Nr. 901, S. 383),
- Acridinorange (Nr. 908, S. 387) und
- Trypaflavin (Nr. 906, S. 386);

Chinolinfarbstoffe, wie
- Pinacyanol (Nr. 924, S. 396) und
- Kryptocyanin (Nr. 927, S. 397);

Chinonfarbstoffe und Ketonfarbstoffe, wie
- Alizarin (Nr. 1 141, S. 449),
- Alizarinrot S (Nr. 1 145, S. 502) und
- Chinizarin (Nr. 1 148, S. 504);

Cyaninfarbstoffe.

Sensibilisierungsfarbstoffe der genannten Art sind in DE-PS 26 08 082 (entsprechend US-PS 4 218 247) ausführlich beschrieben.

Es ist auch bekannt (DE-PS 19 04 629, entsprechend US-PS 3 560 207), elektrophotographisches Aufzeichnungsmaterial mit einem organischen Photoleiter in der photoleitfähigen Schicht zu verwenden, dessen chemische Aktivierung und optische Sensibilisierung über den Bereich des sichtbaren Spektrums durch Zusatz von Cyaninfarbstoffen erfolgt, welche als Substituenten beispielsweise gegebenenfalls substituierte Thiazol-, Oxazol-, Selenazol-, Thiazolin-, Pyridin-, Chinolin-, 3,3-Dialkyl-indolenin-, Imidazol-, Imidazo/4,5-b/chinoxalin-, 3,3-Dialkyl-3H-pyrrol/2,3-b/pyridin- oder Thiazo/4,5-b/chinolin-Gruppen tragen können.

Es ist ferner bekannt (DE-PS-25 26 720, entsprechend US-PS 4 063 948), elektrophotographisches Aufzeichnungsmaterial mit einem elektrisch leitenden Schichtträger zur Herstellung von Druckformen oder gedruckten Schaltungen mit Cyaninfarbstoffen der allgemeinen Formel

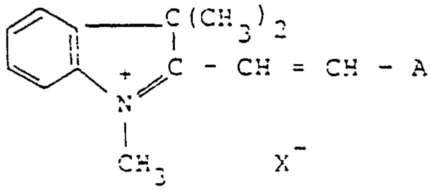

in der

A eine gegebenenfalls substituierte Indolyl-Gruppe, eine gegebenenfalls substituierte Benzthiazolylaminogruppe, eine gegebenenfalls substituierte Phenylaminogruppe oder eine gegebenenfalls substituierte Indolinylgruppe sein kann und

$X^-$ ein einwertiges Anion

bedeutet, zu sensibilisieren.

Die Sensibilität der Photoleiter beruht im Prinzip darauf, daß die im längerwelligen Bereich zur Verfügung stehende Lichtenergie von den Sensibilisierungsfarbstoffen aufgenommen und auf das Photoleitermolekül übertragen wird.

Die hiermit erzielbaren spektralen Sensibilisierungen besitzen jedoch vielfach den Nachteil, daß sie einen sehr breiten Fuß aufweisen, was besonders bei Sensibilisierungsfarbstoffen gemäß US-PS 4 334 001 in Erscheinung tritt, und dadurch nicht dunkelkammersicher sind. Nachteilig hieran ist auch, daß sie zuweilen mehrere Sensibilisierungsmaxima besitzen und/oder das Sensibilisierungsmaximum nicht scharf ausgebildet ist. Oft liegt es auch nicht in dem Gebiet, in dem die zur Exposition benutzte Lichtquelle am stärksten emittiert, was zum Beispiel bei einem Aufzeichnungsmaterial für Laserstrahlbelichtung von entscheidender Wichtigkeit ist. Darüberhinaus wird oftmals die spektrale Lichtempfindlichkeit zwar erhöht, die Allgemeinempfindlichkeit aber vermindert bzw. nicht verbessert. Schließlich färben die verwendeten Sensibilisierungsfarbstoffe vielfach die photoleitfähige Schicht auch stark an, was für manche Anwendungsgebiete hinderlich ist.

Es war deshalb Aufgabe der Erfindung, einen Sensibilisierungsfarbstoff anzugeben, der bei guter Dunkelkammersicherheit mit einem Steilabfall der spektralen Lichtempfindlichkeit nach dem längerwelligen Bereich hin sich durch ein ausgeprägtes Sensibilisierungsmaximum im Bereich von etwa 500 bis 520 nm auszeichnet und einer photoleitfähigen Schicht eine intensive spektrale Lichtempfindlichkeit verleiht.

Die Lösung dieser Aufgabe geht aus von einem elektrophotographischen Aufzeichnungsmaterial, bestehend aus einem elektrisch leitenden Schichtträger und mindestens einer photoleitfähigen Schicht, enthaltend organischen Photoleiter, Sensibilisierungsfarbstoff, Bindemittel und übliche Zusätze, und sie ist dadurch gekennzeichnet, daß die photoleitfähige Schicht als Sensibilisierungsfarbstoff eine Verbindung der allgemeinen Formel

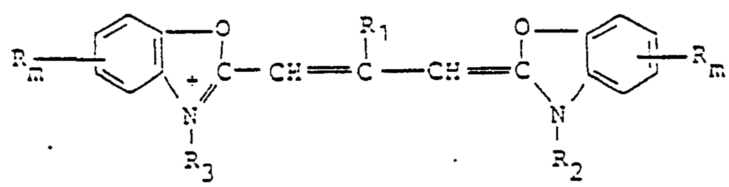

in welcher

R Chlor oder Brom

m 1 oder 2

$R_1$ Alkyl mit 1 bis 3 Kohlenstoffatomen

$R_2$ $-(CH_2)_n-SO_3H$ oder $-(CH_2)_2-COOH$

$R_3$ $-(CH_2)_n-SO_3^-$ oder $-(CH_2)_2-COO^-$ und

n 3 oder 4

bedeuten, enthält. Vorzugsweise enthält die photoleitfähige Schicht als Sensibilisierungsfarbstoff eine Verbindung der folgenden Formel

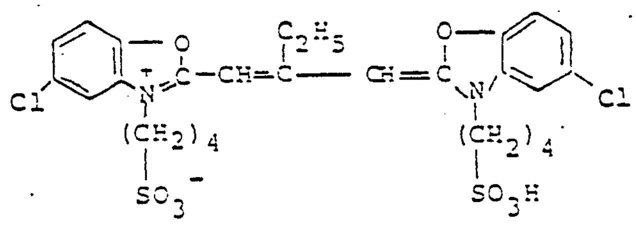

Hierdurch wird erreicht, daß ein gegen grünes, gelbes und rotes Licht dunkelkammersicheres Aufzeichnungsmaterial für die elektrophotographische Reproduktion hergestellt werden kann, das eine scharf ausgeprägte selektive Sensibilisierung mit einem Maximum bei 505 bis 510 nm besitzt, und einen starken Steilabfall des Sensibilisierungsspektrums nach dem längerwelligen Teil des Spektrums aufweist.

Es wird hierdurch auch erreicht, daß nunmehr Aufzeichnungsmaterialien zur Verfügung gestellt werden können, die in Mischung mit anderen selektiv wirkenden Sensibilisierungsfarbstoffen bei guter Verträglichkeit miteinander zwei selektive Sensibilisierungen besitzen, deren Maxima um mehr als 60 nm

auseinander liegen und durch eine Lücke getrennt sind. Außerdem wird erreicht, daß eine panchromatische Sensibilisierung erzielt werden kann, wenn Sensibilisierungsfarbstoffe zugemischt werden, die längerwellig sensibilisieren.

Aus der Photographie ist bekannt, daß viele Cyaninfarbstoffe Silberhalogenid gut spektral zu sensibilisieren vermögen. Dagegen ist die Sensibilisierung elektrophotographischer Aufzeichnungsmaterialien, die organische Photoleiter enthalten, mit derartigen Farbstoffen oft nur gering oder es tritt keine Sensibilisierung ein.

Es war daher überraschend, daß der erfindungsgemäße Sensibilisierungsfarbstoff die photoleitfähige Schicht gut und in selektiver Weise sensibilisiert.

Der vorzugsweise erfindungsgemäß verwendete Sensibilisierungsfarbstoff nach Formel 2 der angefügten Formeltabelle zeichnet sich durch ein intensives und schmales Sensibilisierungsspektrum mit einem scharfen Maximum bei 505 bis 510 nm, einer geringen Sensibilisierungsschulter bei ca. 480 nm und durch einen starken Steilabfall nach dem längerwelligen Bereich hin aus. Dies gewährleistet eine hohe Dunkelkammersicherheit gegen längerwelliges Licht bereits ab ca. 530 nm. Hierdurch erzielt man zusätzlich mehr Sicherheit und eine bessere Qualität der hergestellten Produkte durch visuelle Kontrolle der Arbeitsvorgänge und angenehmere Arbeitsbedingungen. Das Aufzeichnungsmaterial ist dadurch sehr geeignet für Argonionenlaser, die bei 514 nm (488 nm) emittieren, und für im ähnlichen Gebiet emittierende Lichtquellen.

Der erfindungsgemäße Sensibilisierungsfarbstoff bewirkt im Vergleich zu den bekannten Farbstoffen, wie zum Beispiel Kristallviolett oder Rhodamin FB oder Acridinorange, praktisch keine Restfärbung.

Die Herstellung der erfindungsgemäßen Sensibilisierungsfarbstoffe kann nach den Angaben in DE-PS 704 141 bzw. US-PS 2,503,776 oder analog erfolgen.

Wie schon erwähnt, kann der erfindungsgemäße Sensibilisierungsfarbstoff auch in Kombination mit anderen Farbstoffen verwendet werden. Hierdurch kann sich beispielsweise mit einem Sensibilisierungsfarbstoff nach Formel 3 der Formeltabelle eine intensive Mischsensibilisierung bis ca. 600 nm ergeben. Andererseits bewirkt ein Farbstoff der nach Formel 4 angegebenen Struktur, der allein gut und selektiv sensibilisiert, in Mischung mit dem erfindungsgemäßen Sensibilisierungsfarbstoff praktisch keine Sensibilisierung.

Weiterhin können Mischungen von Sensibilisierungsfarbstoffen eingesetzt werden mit Farbstoffen, deren Sensibilisierungsmaximum längerwellig liegt, und die einer anderen chemischen Klasse angehören und mit Silberhalogenid keine oder nur eine geringe Sensibilisierung bewirken, wie z. B. Säuregrün (Acid Green 5, Color Index 42095).

Bei der erfindungsgemäßen Sensibilisierung kann eine eventuell geringere Allgemeinempfindlichkeit im Vergleich zu Systemen, die beispielsweise mit breiter sensibilisierenden Triarylmethanfarbstoffen und nicht selektiven Lichtquellen arbeiten, durch die höhere Lichtintensität der Laserbelichtung und durch eine selektiv wirkende Sensibilisierung kompensiert werden.

Die Konzentration des erfindungsgemäßen Sensibilisierungsfarbstoffes hängt von dem im Einzelfall verwendeten Photoleiter, dem erwünschten Effekt und auch von den verwendeten Sensibilisierungfarbstoffen ab. Üblicherweise werden 0,01 bis 15 Gewichtsprozent, bezogen auf das Gewicht des anwesenden Photoleiters, zugesetzt. Vorzugsweise werden 0,1 bis 1,0 Gewichtsprozent verwendet.

Bei Verwendung eines oder mehrerer selektiv sensibilisierender Farbstoffe ergibt sich dabei die Möglichkeit, beispielsweise bei der Herstellung gedruckter Schaltungen, als Vorlage die Montage einer gedruckten Schaltung auf einem Standbogen zu verwenden, der mit einfarbigen bzw. mehrfarbigen Markierungen versehen ist, die der Lage der jeweiligen Sensibilisierungsmaxima entsprechen. Dadurch werden diese Markierungen oder andere Informationen nicht wiedergegeben. Gleichzeitig kann bei hellem Dunkelkammerlicht gearbeitet werden, das der jeweiligen Sensibilisierungslücke entspricht.

In bekannter Weise erhält man nach Bebilderung, Entwicklung und Entfernen der Photoleiterschicht und Wegätzen der Metallschicht an den belichteten Stellen eine gedruckte Schaltung.

Das Aufzeichnungsmaterial, in dem Ladungserzeugung und Ladungstransport in einer photoleitfähigen Schicht oder in mehreren Schichten, vorzugsweise Doppelschichten, erfolgen können, enthält Photoleiter auf organischer Basis, wie sie im Prinzip bekannt sind. Vorzugsweise werden Oxdiazolderivate, wie 2,5-Bis-(4'-diethylaminophenyl)-1,3,4-oxdiazol, beschrieben in DE-PS 10 58 836 (entsprechend US-PS 3 189 447), oder Oxazolderivate, wie 2-Vinyl-4-(2'-chlorphenyl)-5-(4"-diethylaminophenyl)-oxazol, beschrieben in DE-PS 10 60 260 (entsprechend US-PS 3 112 197) bzw. 11 20 875 (entsprechend US-PS 3 257 203), oder 2-Phenyl-4-(2'-chlorphenyl)-5-(4"-diethylaminophenyl)-oxazol eingesetzt.

Als Bindemittel sind hinsichtlich der Filmbildungseigenschaften und der Haftfestigkeit bekannte Natur- bzw. Kunstharze geeignet. Bei ihrer Auswahl spielen außer den filmbildenden und elektrischen Eigenschaften sowie denen der Haftfestigkeit auf dem Schichtträger auch Löslichkeitseigenschaften eine Rolle. Für Druckzwecke sind solche Bindemittel besonders geeignet, die in wäßrigen oder alkoholischen Lösungsmittelsystemen, gegebenenfalls unter Säure- oder Alkalizusatz, löslich sind. Aus physiologischen und Sicherheitsgründen scheiden aromatische oder aliphatische, leicht brennbare Lösungsmittel aus. Geeignete Bindemittel sind hiernach hochmolekulare Substanzen, die alkalilöslich machende Gruppen tragen, wie Säureanhydrid-, Carboxyl-, Phenol-, Sulfosäure-, Sulfonamid- oder Sulfonimidgruppen. Mischpolymerisate mit Anhydridgruppen können mit besonders gutem Erfolg verwendet werden, da durch das Fehlen freier Säuregruppen die Dunkelleitfähigkeit der photoleitfähigen Schicht gering ist trotz

guter Alkalilöslichkeit.

Die Schichtträger des Aufzeichnungsmaterials können flächig oder zylindrisch ausgebildet sein und in bekannter Weise aus einer Metallplatte, einer Metallfolie, aus metallisierten Papieren oder metallisierten Folien, aus elektrisch leitenden Papieren oder aus Papieren oder Folien, die mit einem elektrisch leitenden Kunststoff beschichtet sind, bestehen.

Auf dem erfindungsgemäßen Aufzeichnungsmaterial können in bekannter Weise auf diesem selbst Tonerbilder erzeugt werden, es ist aber auch möglich, entweder das Ladungsbild oder Tonerbild auf ein Bildempfangsmaterial zu übertragen.

Nachstehende Beispiele sollen die Erfindung näher erläutern, jedoch nicht hierauf begrenzen :

Beispiel 1

Eine Lösung von 10 g 2-Phenyl-4-(2′-chlorphenyl)-5-(4″-diethylaminophenyl)-oxazol, 15 g eines Mischpolymerisates aus Styrol und Maleinsäureanhydrid mit einem Erweichungspunkt von 210 °C, 116 g Tetrahydrofuran, 33 g Butylacetat, 76 g Methylglykol, 50 mg (= 0,5 %, bezogen auf das Gewicht des Photoleiters) des in der Formeltabelle unter Nr. 2 angeführten Chlorbenzoxazol-trimethincyanin-Sensibilisatorfarbstoffes mit einem Absorptionsspektrum (gemessen in alkoholischer Lösung, 10 mg Farbstoff/1, Durchmesser 1 cm) gemäß Figur 3 wurde auf eine oberflächlich elektrochemisch aufgerauhte und anodisierte, 100 µm starke Aluminiumfolie aufgebracht. Nach dem Verdunsten des Lösungsmittels hinterblieb eine ca. 5 µm dicke photoleitfähige Schicht.

Die Schicht wurde mittels einer Corona (Spannung 5 kV negativ, Abstand 25 mm) aufgeladen und mit einem Argonionenlaser im Leistungsbereich 0,2 bis 0,5 mW (Nennleistung 50 mW, Ausgangsleistung 15 mW) bei einem Vorschub von 400 Linien/cm in einem üblichen Automaten, der zur Herstellung von Druckformen dient, bebildert und dann mit einem elektrostatischen Trockentoner handelsüblicher Art entwickelt.

Die Druckplatte ist mit einem Maximum bei 505 bis 510 nm selektiv und mit Steilabfall zum langwelligen Gebiet des Spektrums hin spektral empfindlich, wie dies in Figur 1 zum Ausdruck kommt, und zeichnet sich durch hohe Dunkelkammerlichtsicherheit aus.

Vergleichsweise wurde ein Aufzeichnungsmaterial hergestellt und bebildert mit einer photoleitfähigen Schicht, die anstelle des erfindungsgemäßen Sensibilisatorfarbstoffes eine vergleichbare Menge an Astrazonorange R (C.I. 48 040 — Cyaninfarbstoff) enthielt. Die spektrale Sensibilisierung ist in Figur 2, das Absorptionsspektrum in Figur 4 angegeben. Die Spektrogramme wurden durch Belichtung mit einer Xenonhochdrucklampe XBO 150 W/1 durch ein Interferenzverlauffilter VERIL B-60, Nr. B 70776.47 mit Graukeil und Tonerentwicklung erhalten. Aus dem Vergleich der Figuren 1, 2, 3 und 4 geht hervor, daß die Sensibilisierung nach dem Vergleichsmuster weniger selektiv und der Steilabfall auf der längerwelligen Seite und damit die Dunkelkammerlichtsicherheit weniger stark ausgeprägt ist gegenüber den Daten nach dem erfindungsgemäßen Aufzeichnungsmaterial.

Die spektrale Empfindlichkeit in $cm^2/Ws$ beträgt bei Belichtung mit einem Kryptonlaser an der langwelligen Kante des Sensibilisierungsspektrums für den erfindungsgemäßen Sensibilisierungsfarbstoff (in Klammern die Werte für Astrazonorange R ; Spektrogramme Abb. 2 und 4) bei 521 nm = 4 300 (8 000), 531 nm = 2 100 (6 500), 545 nm (mit Monochromator) = 500 (2 500).

Beispiel 2

Eine Lösung von 15 g 2,5-Bis-(4′-diethylaminophenyl)-1,3, 4-oxdiazol, 15 g eines Mischpolymerisates aus Styrol und Maleinsäureanhydrid, wie in Beispiel 1, 139 g Tetrahydrofuran, 40 g Butylacetat, 91 g Methylglykol, 30 mg des in Beispiel 1 genannten Sensibilisierungsfarbstoffes und 75 mg des Trimethincyaninfarbstoffes nach Formel 3 wurde auf eine aluminiumbedampfte, 100 µm dicke Polyesterfolie aufgebracht. Nach dem Verdunsten des Lösungsmittels resultierte eine etwa 5 µm dicke photoleitfähige Schicht mit einer spektralen Empfindlichkeit vom kurzwelligen sichtbaren Gebiet bis etwa 600 nm, wie dies aus Figur 5 hervorgeht.

Die Folie wurde mit einer Corona auf etwa — 450 V aufgeladen und in einer Reprokamera mit 8 Autophotlampen zu je 500 Watt 25 Sekunden lang belichtet. Als Vorlage diente die Montage einer gedruckten Schaltung auf einem Standbogen, der mit blauen und grün-gelben Markierungen und Orientierungslinien versehen war. Durch die Lichtempfindlichkeit der Photoleiterschicht in diesem Spektralbereich werden die Markierungen auf der kopierten Folie nicht wiedergegeben. Nach dem Entwickeln mit einem elektrophotographischen Entwickler und Entfernen der Photoleiterschicht an den Nichtbildstellen nach dem in der DE-PS 23 22 047 (entsprechend US-PS 4,066,453) beschriebenen Verfahren wird die freigelegte aufgedampfte Aluminiumschicht durch Behandeln mit 2 n Natronlauge entfernt. Man erhält auf diese Weise eine gedruckte Schaltung.

Beispiel 3

Zu einer Lösung aus 8 g 2-Vinyl-4-(2′-chlorphenyl)-5-(4″-diethylaminophenyl)-oxazol und 18 g eines Mischpolymerisates aus Styrol und Maleinsäureanhydrid, wie in Beispiel 1, in einer Mischung aus 90 g

Methylglykol, 140 g Tetrahydrofuran und 40 g Butylacetat, 85 %ig, werden 24 mg des in der Formeltabelle unter Nr. 2 angegebenen Sensibilisatorfarbstoffes, 40 mg des Sensibilisatorfarbstoffes nach Formel 3 und 64 mg Säuregrün (C.I. 42 095, Acid Green 5) zugesetzt. Die Lösung wird auf eine oberflächlich elektrochemisch aufgerauhte und anodisierte Aluminiumfolie aufgebracht, die auf ihrer Oberfläche, wie in DE-OS 16 21 478 (entsprechend US-PS 4,153,461) beschrieben, mit Polyvinylphosphonsäure vorbehandelt wurde. Nach dem Verdunsten des Lösungsmittels erhält man eine vom kurzwelligen, sichtbaren Gebiet des Spektrums bis ca. 660 nm lichtempfindliche Schicht, die einerseits die verwendete Lichtquelle gut ausnutzt, andererseits aber auch gestattet, noch im sichtbaren Rotlicht durch visuelle Kontrolle die Arbeitsvorgänge zu beobachten (Fig. 6).

Aus diesem Aufzeichnungsmaterial wird auf folgende Weise eine Druckform für den Offsetdruck hergestellt. Man lädt die photoleitfähige Schicht im Dunkeln mit Hilfe einer Corona auf — 430 V auf und belichtet sie in einer Reprokamera bei Blende 14 zehn Sekunden, wobei als Lichtquelle 10 MH-Strahler von je 600 W Leistung verwendet werden. Das entstandene latente Ladungsbild wird mit einem handelsüblichen Trockentoner mit Hilfe einer Magnetwalze entwickelt und das Tonerbild durch Wärme fixiert. Nach Entfernen der photoleitfähigen Schicht an den nicht mit Toner bedeckten Stellen mit einer Lösung, die man dadurch erhält, daß man 50 g $Na_2SiO_3 \cdot 9\ H_2O$ in 250 g Glycerin (86 %ig) löst und mit 390 g Ethylenglykol und 310 g Methanol verdünnt, erhält man eine Flachdruckform, mit der in hoher Auflage gedruckt werden kann.

**Patentansprüche**

1. Elektrophotographisches Aufzeichnungsmaterial, bestehend aus einem elektrisch leitenden Schichtträger und mindestens einer photoleitfähigen Schicht, enthaltend organischen Photoleiter, Sensibilisierungsfarbstoff, Bindemittel und übliche Zusätze, dadurch gekennzeichnet, daß die photoleitfähige Schicht als Sensibilisierungsfarbstoff eine Verbindung der allgemeinen Formel

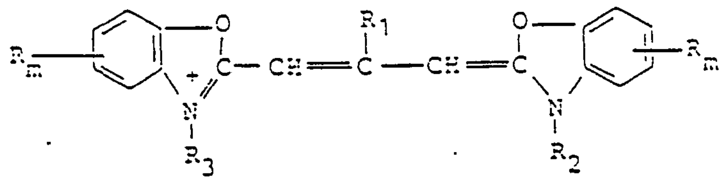

in welcher
R Chlor oder Brom
m 1 oder 2
$R_1$ Alkyl mit 1 bis 3 Kohlenstoffatomen
$R_2$ $-(CH_2)_n-SO_3H$ oder $-(CH_2)_2-COOH$
$R_3$ $-(CH_2)_n-SO_3^-$ oder $-(CH_2)2-COO^-$ und
n 3 oder 4
bedeuten, enthält.

2. Aufzeichnungsmaterial nach Anspruch 1, dadurch gekennzeichnet, daß die photoleitfähige Schicht als Sensibilisierungsfarbstoff eine Verbindung der folgenden Formel

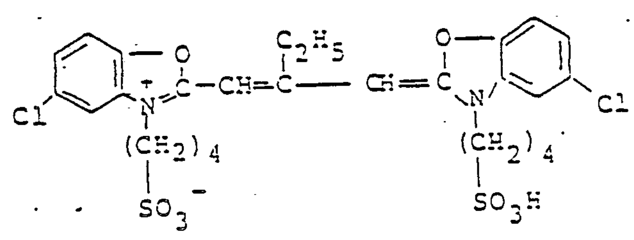

enthält.

3. Aufzeichnungsmaterial nach Ansprüchen 1 oder 2, dadurch gekennzeichnet, daß die photoleitfähige Schicht zusätzlich einen Sensibilisierungsfarbstoff mit einem Sensibilisierungsmaximum, das um mehr als 60 nm im längerwelligen Bereich liegt, enthält,

4. Aufzeichnungsmaterial nach Anspruch 3, dadurch gekennzeichnet, daß die photoleitfähige Schicht als zusätzlichen Sensibilisierungsfarbstoff eine Verbindung der Formel

(Siehe Formel Seite 7 f.)

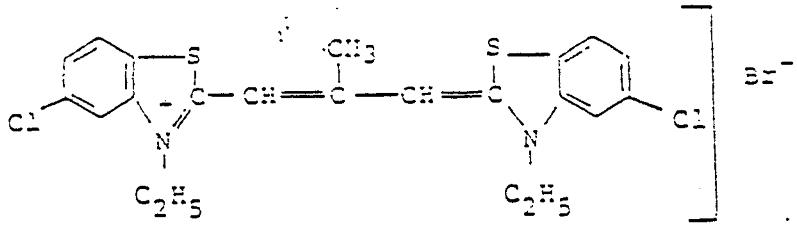

enthält.

5. Aufzeichnungsmaterial nach Ansprüchen 1 bis 4, dadurch gekennzeichnet, daß die photoleitfähige Schicht zusätzlich als Sensibilisierungsfarbstoff Säuregrün (Acid Green 5, C.I. 42095) enthält.

6. Aufzeichnungsmaterial nach Anspruch 1, dadurch gekennzeichnet, daß die photoleitfähige Schicht als organischen Photoleiter Verbindungen auf Basis von Oxazol- oder Oxdiazolderivaten enthält.

**Claims**

1. Electrophotographic recording material comprising an electrically conductive support and at least one photoconductive layer which contains an organic photoconductor, a sensitizing dye, a binder, and customary additives, wherein the sensitizing dye contained in the photoconductive layer comprises a compound corresponding to the general formula

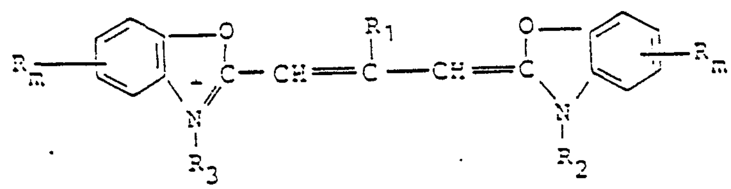

wherein

R is chlorine or bromine

m is 1 or 2,

$R_1$ is an alkyl group having from 1 to 3 carbon atoms,

$R_2$ is $-(CH_2)_n-SO_3H$ or $-(CH_2)_2-COOH$,

$R_3$ is $-(CH_2)_n-SO_3^-$ or $-(CH_2)_2-COO^-$ and

n is 3 or 4.

2. A recording material as claimed in claim 1, wherein the sensitizing dye contained in the photoconductive layer comprises a compound corresponding to the following formula :

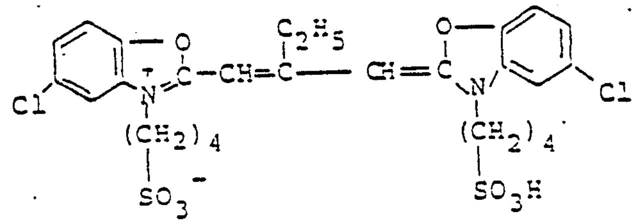

3. A recording material as claimed in claim 1 or claim 2, wherein the photoconductive layer additionally contains a sensitizing dye having a sensitization peak which is by more than 60 nm in the longer-wavelength region.

4. A recording material as claimed in claim 3, wherein the photoconductive layer contains as an additional sensitizing dye a compound corresponding to the formula

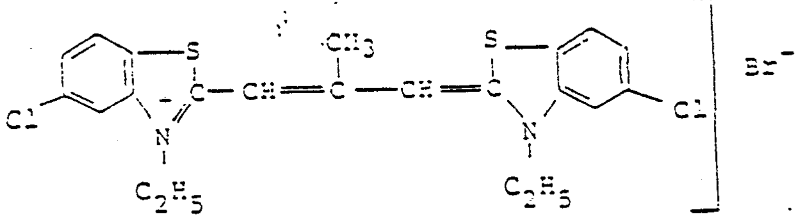

5. A recording material as claimed in claims 1 to 4, wherein the photoconductive layer additionally contains Acid Green (Acid Green 5, C.I. 42095), as a sensitizing dye.

6. A recording material as claimed in claim 1, wherein the organic photoconductors contained in the photoconductive layer comprise compounds on a basis of oxazole derivatives or oxadiazole derivatives.

**Revendications**

1. Matériau d'enregistrement électrophotographique, composé d'un support de couche conducteur de l'électricité et d'au moins une couche photoconductrice contenant un photoconducteur organique, un colorant sensibilisateur, un liant et des additifs usuels, caractérisé en ce que la couche photoconductrice contient en tant que colorant sensibilisateur un composé de formule générale

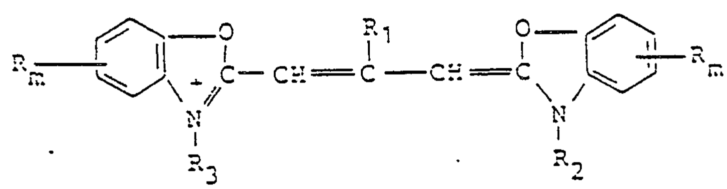

dans laquelle
R représente un atome de chlore ou de brome,
m est 1 ou 2,
$R_1$ représente un groupe alkyle ayant de 1 à 3 atomes de carbone,
$R_2$ représente $-(CH_2)_n-SO_3H$ ou $-(CH_2)_2-COOH$,
$R_3$ représente $-(CH_2)_n-SO_3^-$ ou $-(CH_2)_2-COO^-$ et
n est 3 ou 4.

2. Matériau d'enregistrement selon la revendication 1, caractérisé en ce que la couche photoconductrice contient en tant que colorant sensibilisateur un composé de formule suivante

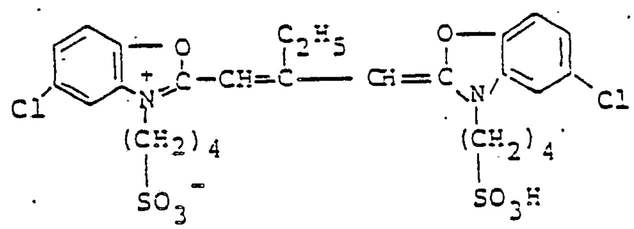

3. Matériau d'enregistrement selon les revendications 1 ou 2, caractérisé en ce que la couche photoconductrice contient en outre un colorant sensibilisateur ayant un maximum de sensibilisation qui se situe à plus de 60 nm à l'intérieur de la zone des longueurs d'onde relativement grandes.

4. Matériau d'enregistrement selon la revendication 3, caractérisé en ce que la couche photoconductrice contient en tant que colorant sensibilisateur supplémentaire un composé de formule

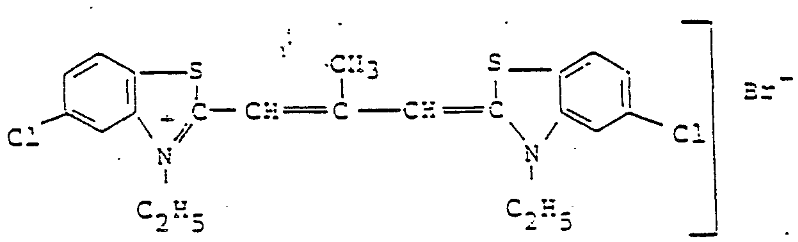

5. Matériau d'enregistrement selon les revendications 1 à 4, caractérisé en ce que la couche photoconductrice contient en outre, en tant que colorant sensibilisateur, du vert acide (Acid Green 5, C.I. 42095).

6. Matériau d'enregistrement selon la revendication 1, caractérisé en ce que la couche photoconductrice contient en tant que photoconducteurs organiques des composés à base de dérivés d'oxazole ou d'oxadiazole.

FORMELTABELLE

1

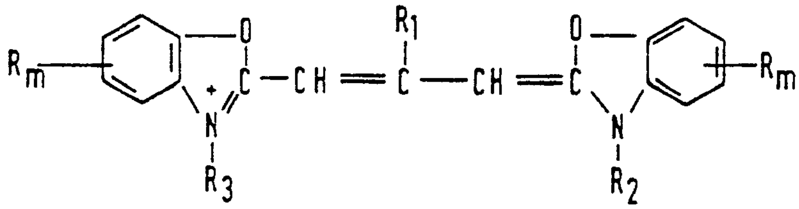

2

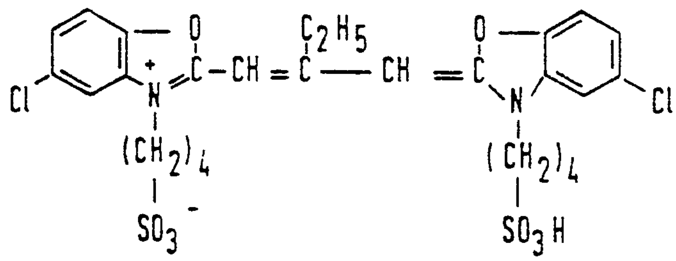

3

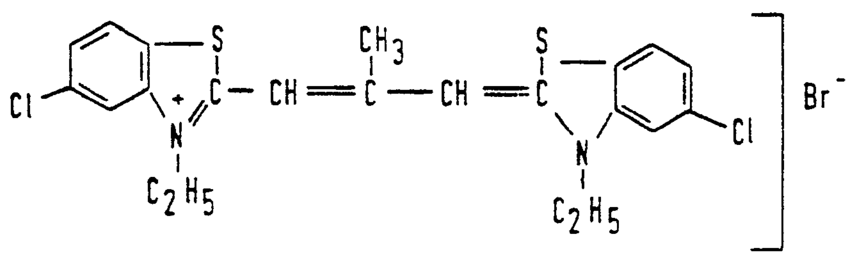

4

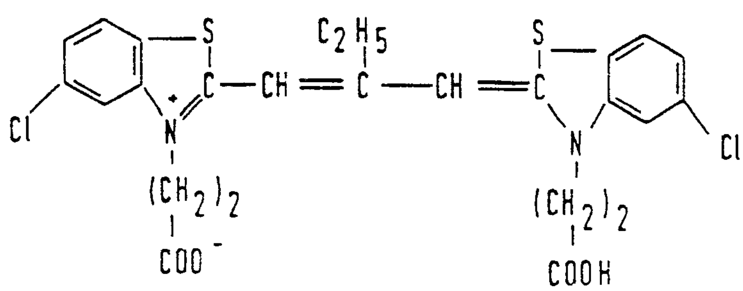

FIG. 1

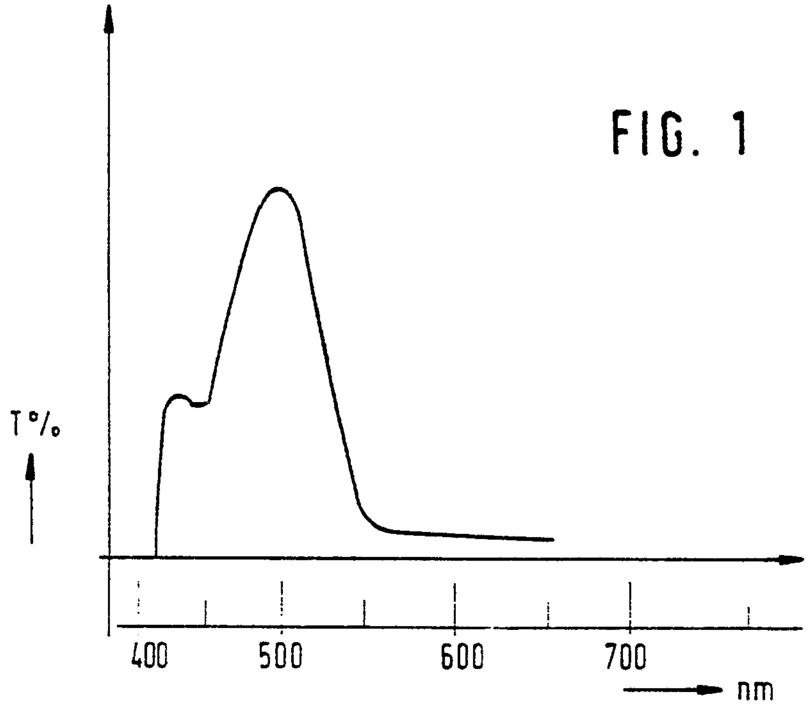


FIG. 2

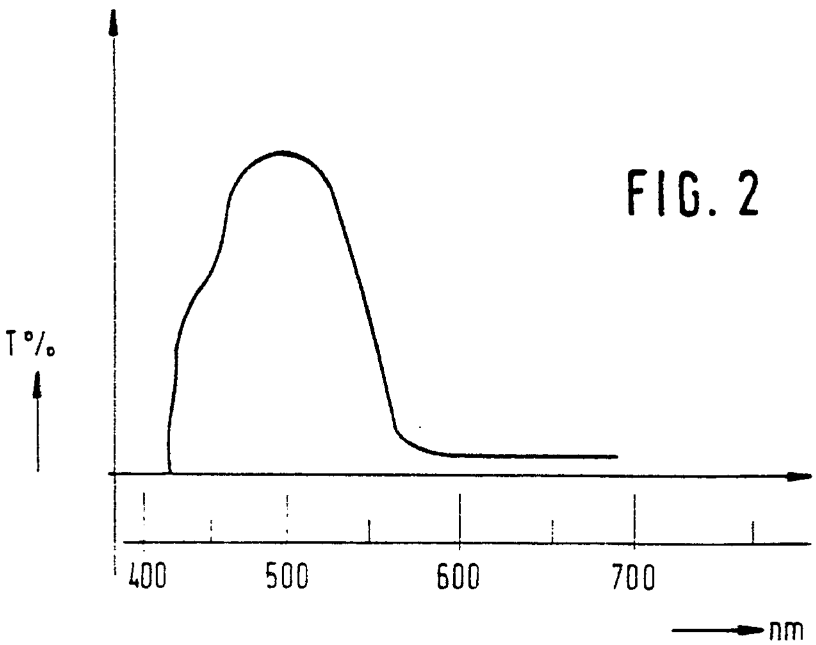

FIG. 4

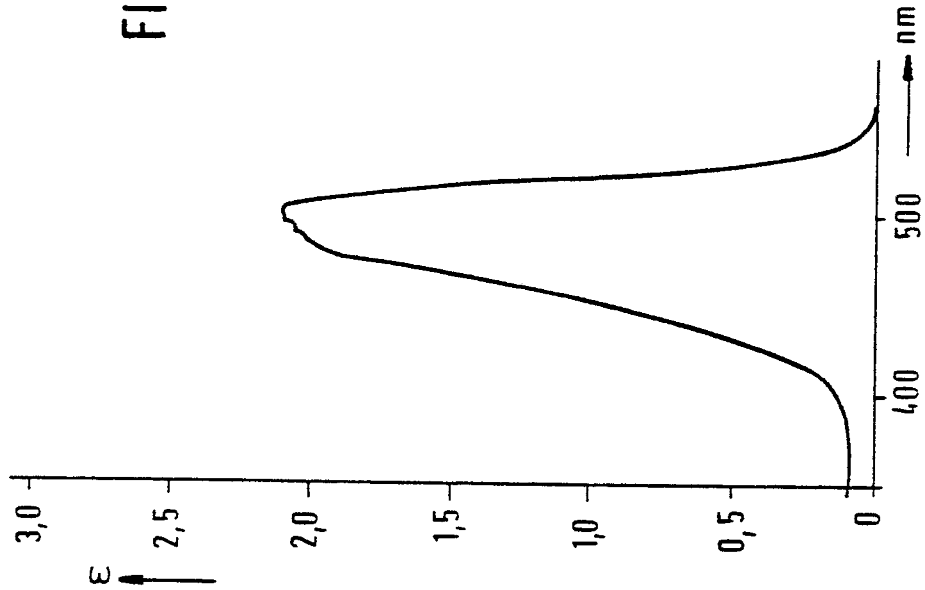


FIG. 3

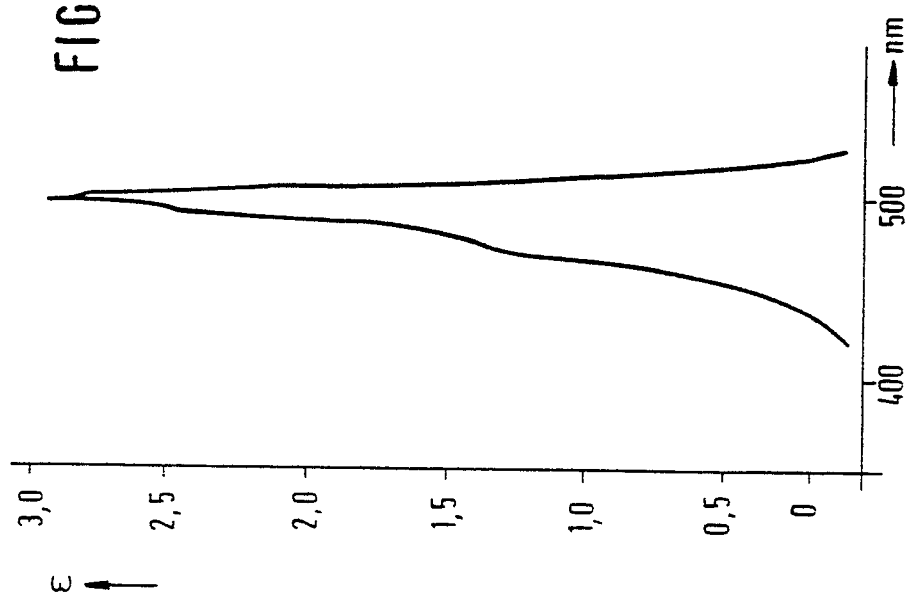

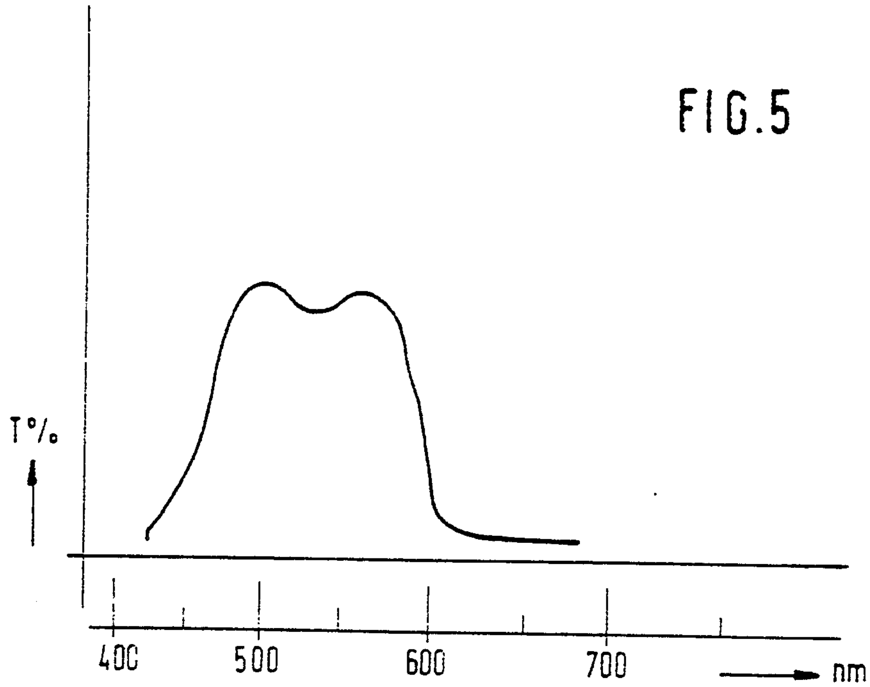


FIG.5

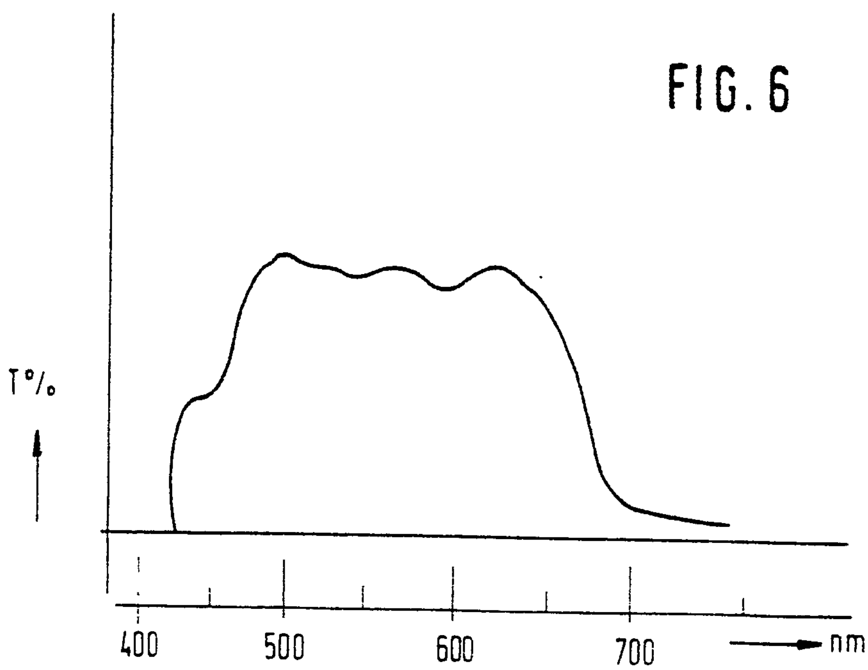


FIG.6